# EUROPÄISCHE PATENTSCHRIFT

(11) **EP 1 815 880 B1**
(45) Veröffentlichungstag und Bekanntmachung des Hinweises auf die Patenterteilung: **09.09.2009**
(21) Anmeldenummer: 07009418.0
(22) Anmeldetag: 26.02.2002
(51) Int. Cl.: A61M 5/32, A61M 25/06

(54) **Schutzvorrichtung für eine Injektionsnadel**
Protection device for an injection needle
Dispositif de protection pour une aiguille d'injection

(30) Priorität: 26.02.2001 DE 20103363 U
(43) Veröffentlichungstag der Anmeldung: 08.08.2007
(62) Teilanmeldung aus: 06013171.1
(73) Patentinhaber: B. BRAUN MELSUNGEN AG, 34212 Melsungen (DE)
(72) Erfinder: Woehr, Kevin, 34587 Felsberg (DE); Fuchs, Jürgen, 34308 Bad Emstal (DE)
(74) Vertreter: Klingseisen, Franz

(56) Entgegenhaltungen:
- EP-A1- 0 713 710
- WO-A-99/08742
- WO-A-99/12592
- US-A- 5 957 892

## Beschreibung

Die Erfindung betrifft eine Schutzvorrichtung nach dem Oberbegriff des Anspruchs 1.

Aus EP-A1-0 713 710 ist eine Schutzvorrichtung dieser Art bekannt, wobei am Nadelhalter eine faltbare Halteeinrichtung mit drei Abschnitten befestigt ist, die am distalen Ende ein etwa zylindrisches Teil mit einem darin angebrachten Nadelschutzelement hält. Wenn die Abschnitte der faltbaren Halteeinrichtung entfaltet sind und sich das Schutzelement in der Schutzstellung befindet, in der ein Arm des Schutzelementes die Nadelspitze übergreift, wird durch die entfaltete Halteeinrichtung verhindert, dass das Nadelschutzelement über die Nadelspitze hinaus verschoben werden kann. Deshalb muss die Länge der entfalteten Halteeinrichtung auf die Nadellänge abgestimmt sein, weil bei einer kürzeren Nadel die Nadelspitze am proximalen Ende des Nadelschutzelementes austreten könnte, wenn die Halteeinrichtung im entfalteten Zustand länger ist als der Nadelschaft zwischen Nadelspitze und Nadelhalter.

Der Erfindung liegt die Aufgabe zugrunde, eine Schutzvorrichtung der eingangs angegebenen Art so auszubilden, dass eine Verletzungsgefahr ausgeschlossen werden kann.

Diese Aufgabe wird erfindungsgemäß durch die Merkmale des Anspruchs 1 gelöst. Die Eingriffseinrichtung zwischen Nadel und Schutzelement verhindert, dass das Nadelschutzelement über die Nadelspitze hinaus verschoben werden kann.

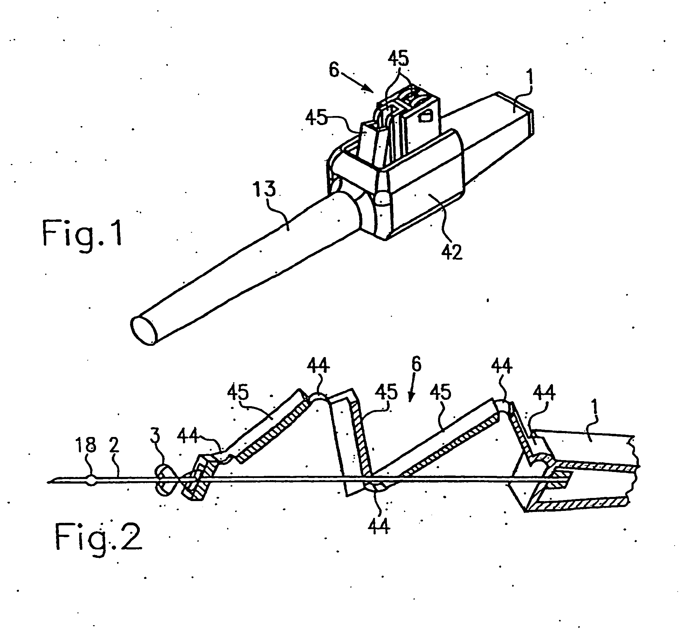

Beispielsweise Ausführungsformen der Erfindung werden nachfolgend anhand der Zeichnungen näher erläutert. Es zeigen
Fig.1 eine Ausführungsform eines verformbaren Griffteils in der Ausgangsstellung,
Fig.2 das Griffteil von Fig. 1 in der ausgefahrenen Stellung, und
Fig.3 einen Längsschnitt durch eine Nadelkappe.
Fig.4 zeigt einen Nadelhalter 1, in dem eine Nadel 2 befestigt ist. Auf dem Schaft der Nadel 2 ist ein Schutzelement 3 in Form eines Federclips mit sich kreuzenden Armen angeordnet, wobei
   das Schutzelement 3 nicht über die Nadelspitze hinaus verschoben werden kann. Fig. 1 und 2 zeigen eine Ausführungsform eines verformbaren Griffteils 6, wobei Fig. 1 das Griffteil im zusammengefalteten Zustand in der Bereitstellung wiedergibt. Eine Nadelkappe 13 ist am proximalen Ende mit einem Aufnahmeabschnitt 42 versehen, der zusammengefaltete Abschnitte 45 des Griffteils 6 aufnimmt, das zwischen Nadelhalter 1 und dem in Fig. 1 nicht dargestellten, im Aufnahmeabschnitt 42 angeordneten Schutzelement 3 angeordnet ist.

Fig. 2 zeigt in schematischer Darstellung das Griffteil 6 in teilweise ausgefahrenem Zustand, nachdem die Nadelkappe 13 abgenommen ist und eine Injektion ausgeführt wurde. Hierauf werden die über Gelenk- bzw. Scharnierabschnitte 44 miteinander verbundenen steifen Abschnitte 45 des Griffteils 6, die teilweise auf der Nadel 2 geführt sind, längs der Nadel verschoben und ausgerichtet, wobei das Schutzelement 3 nach vorne zur Nadelspitze verschoben wird, bis es mit der Nadelkröpfung 18 in Eingriff tritt und die Nadelspitze abdeckt.

Die Ausführungsform nach den Fig. 1 und 2 ist vorteilhaft hinsichtlich der Länge der zur Verfügung stehenden Kanüle , weil durch die Faltung der Abschnitte 45 eine kompaktere Anordnung möglich ist, wie Fig. 1 zeigt. Anstelle der Faltabschnitte in Fig. 2 kann auch ein Scherenmechanismus zwischen Schutzelement und Nadelhalter vorgesehen werden, um auf engem Raum Elemente unterzubringen, mittels denen das Schutzelement weit ausfahrbar ist.

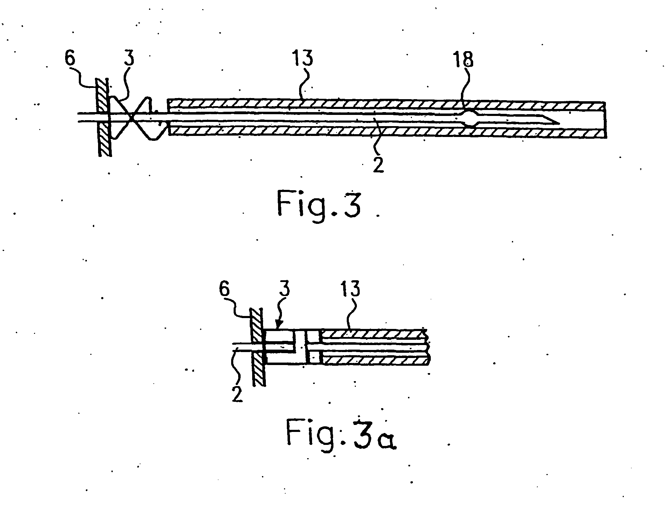

Es wird als Schutzelement 3 bevorzugt ein Nadelclip aus Metall verwendet, dessen sich kreuzende Arme von gegenüberliegenden Seiten eines proximalen Wandabschnitts ausgehen, der ein Loch für den Durchtritt der Nadel aufweist, wobei der Lochdurchmesser kleiner ist als die maximale Querabmessung der Nadel an der Quetschung 18, so dass der Nadelclip durch den im Durchmesser vergrößerten Abschnitt 18 in der Schutzstellung an der Nadelspitze gehalten wird. Die sich kreuzenden Arme, die beiderseits der Nadel 2 verlaufen, wie Fig. 3a zeigt, weisen am distalen Ende einen etwa auf die Breite der Rückwand verbreiterten Endabschnitt auf, der in der Ausgangsstellung auf dem Außenumfang der Nadel unter elastischer Vorspannung aufliegt und bei Erreichen der Nadelspitze durch Federwirkung in die Schutzstellung bewegt wird, in der die beiden verbreiterten Endabschnitte die Nadelspitze übergreifen. Hierzu sind die distalen Enden der Arme, wie die Seitenansichten zeigen, in Längsrichtung etwas versetzt zueinander bzw. die Arme unterschiedlich lang, so dass sichergestellt ist, dass die beiden abgewinkelten Endabschnitte der Arme die Nadelspitze übergreifen. Zumindest am längeren Arm ist der Endabschnitt am freien Rand nach innen gebogen, um die Abdeckung der Nadelspitze auch dann zu gewährleisten, wenn versucht weiden sollte, den Nadelclip aus der Schutzstellung auf der Nadel zurückzuschieben, wobei sich der nach innen abgebogene Endabschnitt an der Nadelspitze verhakt. Der Nadelclip kann insgesamt sehr kompakt ausgebildet werden und nur etwa 7 mm lang sein.

Fig. 3 zeigt einen Längsschnitt durch eine Nadelkappe 13, deren proximales Ende am Schutzelement 3 anliegt. Die Nadelkappe ist rohrförmig ausgebildet, wobei die durch Quetschung ausgebildete Durchmesservergrößerung 18 an der Nadel 2 als Abstandhalter für die Nadelkappe 13 dient. Eine solche Nadelkappe kann durch Extrudieren oder Spritzgießen hergestellt werden. Es ist auch möglich, auf dem Innenumfang der Nadelkappe einen Wulst oder Noppen anzuformen, die am Nadelschaft aufliegen und die Nadel im Wesentlichen konzentrisch in der Nadelkappe führen. Die Nadelkappe 13 wird hierbei durch Reibung an den Vorwölbungen 18 auf der Nadel 2 gehalten.

Nach einer weiteren Ausgestaltung kann die Nadelkappe nach dem Aufstecken auf die Nadel durch Wärme und Druck bzw. durch Schrumpfen auf der Nadel festgelegt werden.

## Patentansprüche

1. Schutzvorrichtung für eine Injektions- oder Infusionsnadel (2), umfassend
einen Nadelhalter (1) am proximalen Ende der Nadel,
ein Schutzelement (3) für die Nadelspitze, das auf dem Schaft der Nadel verschiebbar ist,
wobei dieses Schutzelement durch eine Halteeinrichtung daran gehindert ist, über die Nadelspitze hinaus bewegt zu werden, und
ein Griffteil (6) zwischen Schutzelement (3) und Nadelhalter (1) zum Verschieben bzw. Halten des Schutzelementes,
wobei das Schutzelement relativ zur Nadel zwischen einer Bereitstellung und einer Schutzstellung verschiebbar ist, und
die Schutzvorrichtung weiterhin steife Abschnitte (45) umfasst, wobei ein Abschnitt relativ zum Griffteil verschwenkbar mit dem Griffteil (6) verbunden ist, und wobei Druck auf einen steifen Abschnitt diesen dazu veranlasst, um seine Verbindungsstelle zu schwenken und das Schutzelement relativ zur Nadel aus der Bereitstellung in die Schutzstellung zu verschieben,
**dadurch gekennzeichnet;**
**dass** die Halteeinrichtung als radialer Vorsprung (18) an der Nadel proximal von der Nadelspitze ausgebildet ist, der das Schutzelement daran hindert, über die Nadelspitze hinaus verschoben zu werden.

2. Schutzvorrichtung nach Anspruch 1, wobei das Schutzelement eine Öffnung für den Eingriff mit dem radialen Vorsprung (18) an der Nadel aufweist.

3. Schutzvorrichtung nach einem der vorhergehenden Ansprüche, wobei das Schutzelement als Metallclip ausgebildet ist.

4. Schutzvorrichtung nach Anspruch 3, wobei der Metallclip sich kreuzende Arme aufweist.

5. Schutzelement nach Anspruch 4, wobei die sich kreuzenden Arme unterschiedlich lang sind.

6. Schutzvorrichtung nach einem der vorhergehenden Ansprüche, wobei das Griffteil (6) einen hohlzylindrischen Abschnitt aufweist, der die Finger des Benutzers daran hindert, mit dem Nadelschaft in Berührung zu kommen, wenn das Griffteil längs des madelsdraftes bewegt wird.

7. Schutzvorrichtung nach Anspruch 6, wobei der hohlzylindrische Abschnitt die Spitze der Nadel aufnimmt, wenn das Griffteil im distale Richtung auf der nadel bewegt ist und die Bewegung der Nadel durch die Eingriffseinrichtung zwischen Nadel und Schutzelement begrenzt ist.

8. Schutzvorrichtung nach einem der vorhergehenden Ansprüche, wobei das Schutzelement (3) distal von dem steifen Abschnitt (45) positioniert ist.

9. Nadelaufbau mit einer Schutzvorrichtung nach einem der vorhergehenden Ansprüche.

## Claims

1. Protective device for an injection or infusion needle (2), comprising
a needle holder (1) at the proximal end of the needle,
a protective element (3) for the needle tip, which is displaceable on the shaft of the needle,
wherein this protective element is prevented by a retaining means from being displaced beyond the needle tip, and
a grip part (6) between the protective element (3) and the needle holder (1) for displacing and retaining the protective element,
wherein the protective element is displaceable relative to the needle between a standby position and a protective position, and
the protective device further comprises stiff portions (45), wherein one portion is connected with the grip part (6) and pivotal relative to the grip part (6), and wherein pressure applied to a stiff portion causes the stiff portion to pivot about its connection and to displace the protective element out of the ready position into the protective position relative to the needle,
**characterized in that**
the retaining means is formed as a radial projection (18) at the needle proximally from the needle tip, preventing the protective element from being displaced past the needle tip.

2. The device according to claim 1, wherein the protective element has an opening for engaging with the radial projection (18) on the needle.

3. The device according to any preceding claim, wherein the protective element is formed as a metal clip.

4. The device according to claim 3, wherein the metal clip comprises intersecting arms.

5. The device according to claim 4, wherein the intersecting arms are of different lengths.

6. The device according to any preceding claim, wherein the grip part (6) has a hollow cylindrical portion which prevents the fingers of the user from coming into contact with the needle shaft when the grip part is moved along the needle shaft.

7. The device according to claim 6, wherein the hollow cylindrical portion receives the tip of the needle when the grip part is moved on the needle in the distal direction and the movement of the needle is limited by the engaging means between the needle and the protective element.

8. The device according to any preceding claim, wherein the protective element (3) is positioned distally of the stiff portion (45).

9. A needle assembly comprising a protective device according to any preceding claim.

## Revendications

1. Un dispositif protecteur pour une aiguille (2) pour injection ou infusion, comprenant un porte-aiguille (1) au bout proximale de l'aiguille,
un élément protecteur (3) pour la pointe de l'aiguille, qui est déplaçable sur le manche de l'aiguille,
cet élément protecteur étant empêché par un moyen de rétention d'être déplaçé au-dela de la pointe de l'aiguille, et
une partie poignée (6) entre l'élément protecteur (3) et le porte-aiguille (1) pour déplacer et retenir l'élément protecteur,
l'élément protecteur étant déplaçable relative à l'aiguille entre un position de réserve et une position protecteure, et
l'élément protecteur comprend en outre des sections (45) raides, l'une section étant accouplée avec la partie poignée (6) et pivotante relative à la partie poignée (6), et de la pression appliquée à une section raide faisant pivoter la section raide autour de sa connexion pour déplacer l'élément protecteur hors de la position prête à la position protecteure relative à l'aiguille,
**caractérisé en ce que**
le moyen de rétention est formé comme une saillie radiale (18) sur l'aiguille proximalement de la pointe de l'aiguille, empêchant l'élément protecteur d'être déplaçé au-dela de la pointe de l'aiguille.

2. Le dispositif selon la revendication 1, l'élément protecteur ayant une aperture pour s'enclencher avec la saillie radiale (18) sur l'aiguille.

3. Le dispositif selon une des revendications précédentes, l'élément protecteur étant formé comme une pince métallique.

4. Le dispositif selon la revendication 3, la pince métallique comprenant des bras s'entrecroisants.

5. Le dispositif selon la revendication 4, les bras s'entrecroisants ayant des longueurs différentes.

6. Le dispositif selon une des revendications précédentes, la partie poignée (6) ayant une section creuse cylindrique qui empêche les doigts de l'opérateur de contacter le manche de l'aiguille quand la partie poignée est déplaçée sur le manche de l'aiguille.

7. Le dispositif selon la revendication 6, la section creuse cylindrique abritant la pointe de l'aiguille quand la partie poignée est déplaçée sur l'aiguille dans la direction distale et le mouvement de l'aiguille est limitée par le moyen d'enclenchement entre l'aiguille et l'élément protecteur.

8. Le dispositif selon une des revendications précédentes, l'élément protecteur (3) étant positionné distalement de la section raide (45).

9. Un assemblage d'aiguille comprenant un dispositif protecteur selon une des revendications précédentes.
